# EUROPEAN PATENT APPLICATION

(11) **EP 2 529 777 A1**
(43) Date of publication of application: **05.12.2012**
(21) Application number: 11168015.3
(22) Date of filing: 30.05.2011
(51) Int. Cl.: A61M 5/32

(54) **Needle assembly removal device**

(71) Applicant: Sanofi-Aventis Deutschland GmbH, 65929 Frankfurt (DE)
(72) Inventor: The designation of the inventor has not yet been filed

(57) **Abstract**

Described is a needle assembly removal device comprising an inner portion (10), a channel (12) formed in the inner portion (10), and an outer portion (15) movably disposed on the inner portion (10). The channel (12) has a proximal opening (11). Movement of the outer portion (15) relative to the inner portion (10) in a first direction causes the inner portion (10) to change the proximal opening (11) from a first width to a second width.

## Description

### Technical Field

The invention relates to a needle assembly removal device for removing a needle assembly from an injection device such as, for example, a pen injector or an auto-injector.

### Background of the Invention

Patients suffering from diseases like diabetes have to frequently self-administer injections. Injection devices like auto-injectors or pen injectors have been developed to facilitate self-administering injections. Typically, such injection devices are re-usable and refitted with sterile injection needle assemblies to minimize the risk of infections.

A conventional needle assembly has a needle hub which carries a needle and is connected to an injection device. For re-use of the injection device, the needle assembly has to be removed from the injection device and replaced with an unused needle assembly. The process of removing a used needle assembly from the injection device can involve the risk of needlestick injury if the patient is required to manually remove and/or contact the used needle assembly. Thus, there is a need for a needle assembly removal device.

### Summary of the Invention

It is an object of the present invention to provide a needle assembly removal device for safely removing a needle assembly from an injection device.

In an exemplary embodiment, a needle assembly removal device comprises an inner portion, a channel formed in the inner portion, and an outer portion movably disposed on the inner portion. The channel has a proximal opening. Movement of the outer portion relative to the inner portion in a first direction causes the inner portion to change the proximal opening from a first width to a second width.

In an exemplary embodiment, the first width is greater than a diameter of a needle hub of a needle assembly. The second width is substantially equal to the diameter of the needle hub of the needle assembly.

In an exemplary embodiment, the inner portion includes a first angled surface and the outer portion includes a second angled surface. The first angled surface abuts the second angled surface.

In an exemplary embodiment, movement of the outer portion relative to the inner portion in a second direction causes the inner portion to change the proximal opening from the second width to the first width.

In an exemplary embodiment, the channel includes a distal opening. A third width of the distal opening is equal to or greater than the first width.

In an exemplary embodiment, a spring is coupled to the inner portion. The spring is compressed during movement of the outer portion relative to the inner portion in the first direction.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given hereinbelow and the accompanying drawings which are given by way of illustration only, and thus, are not limitive of the present invention, and wherein:
- Figure 1: shows a schematic sectional view of a needle assembly removal device according to an exemplary embodiment of the present invention,
- Figure 2: shows a schematic sectional view of a needle assembly removal device according to an exemplary embodiment of the present invention,
- Figure 3: shows a schematic sectional view of a needle assembly removal device according to an exemplary embodiment of the present invention,
- Figure 4: shows a schematic sectional view of a needle assembly removal device according to another exemplary embodiment of the present invention,
- Figure 5: shows a schematic sectional view of a needle assembly removal device according to another exemplary embodiment of the present invention, and
- Figure 6: shows a schematic sectional view of a needle assembly removal device according to another exemplary embodiment of the present invention.

Corresponding parts are marked with the same reference symbols in all figures.

### Detailed Description

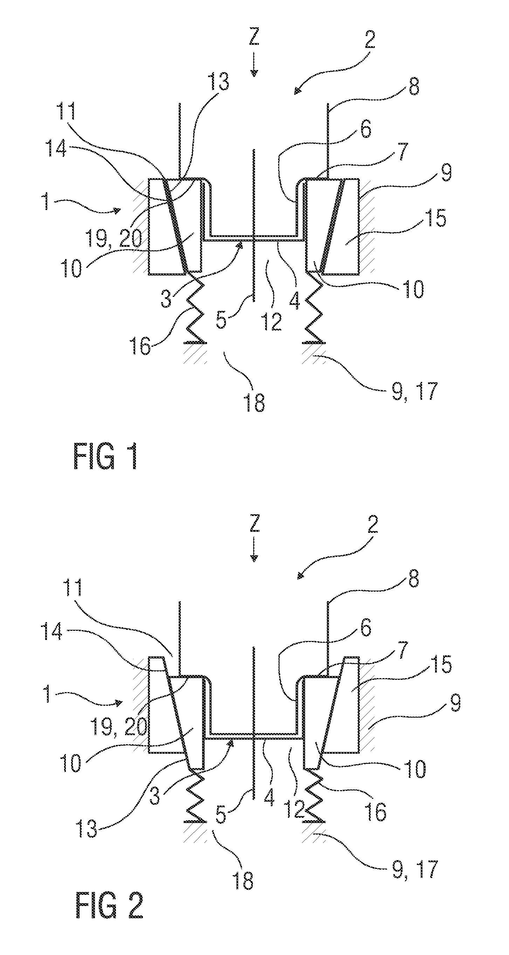

Figures 1 to 3 show a schematic sectional view of an exemplary embodiment of a needle assembly removal device 1 according to the present invention.

As shown in the exemplary embodiment of Figure 1, the needle assembly removal device 1 is utilized to remove a needle assembly 3 which has been coupled to an injection device 2. The needle assembly 3 may comprise a needle hub 4 adapted to mate with a distal end of the injection device 2 and a needle 5 coupled to the needle hub 4. The needle 5 may have a proximal portion which is inserted into a medicament cartridge in the injection device 2 and a distal portion for piercing a patient's skin and delivering a medicament. The needle hub 4 may be coupled to the injection device 2 via a threaded fit, a bayonet fit, a snap fit, a friction fit, etc.

In an exemplary embodiment, the distal end of the injection device 2 includes a neck portion 6, a shoulder portion 7 proximal of the neck portion 6, and a body portion 8 proximal of the shoulder portion 7. The body portion 8 may encase a medicament cartridge and have a septum at a distal end thereof, which may be pierced by the proximal portion of the needle 5 when the needle assembly 3 is coupled to the injection device.

In an exemplary embodiment, the needle assembly removal device 1 includes a housing 9 including an inner portion 10 and an outer portion 15. A channel 12 formed in the inner portion may include a proximal opening 11 and a distal opening 18. The proximal opening 11 may be sized and shaped to receive the needle hub 4 of the needle assembly 3. The outer portion 15 of the housing 9 may surround the inner portion 10. The inner portion 10 may be movable relative to the outer portion 15 along an interface. In an exemplary embodiment, the interface may comprise a first angled surface 13 formed on the inner portion 10 and a second angled surface 14 formed on the external portion 15.

In an exemplary embodiment, a spring 16 is coupled to a distal end of the inner portion 10 and a distal end 17 of the housing 9.

As shown in the exemplary embodiment in Figure 1, after an injection has been administered, the needle assembly removal device 1 is placed on the injection device 2 such that the needle assembly 3 is received in the proximal opening 11 of the channel 12.

As shown in the exemplary embodiment in Figure 2, the needle assembly removal device 1 is advanced in a first direction (e.g., proximally) until a proximal end 19 of the inner portion 10 abuts the shoulder portion 7 of the injection device 2. As the needle assembly removal device 1 is advanced further proximally, the spring 16 compresses, and the outer portion 15 moves proximally relative to the inner portion 10. As the outer portion 15 moves proximally relative to the inner portion 10, the outer portion 15 presses the inner portion 10 and a first width of the proximal opening 11 is changed (e.g., reduced) to a second width. When the proximal opening 11 is at the second width, the inner portion 10 engages the needle hub 4 of the needle assembly 3. Due to the angled orientation of the interface, movement of the outer portion 15 in a first direction causes radial movement of the inner portion 10. Due to the frictional engagement of the needle hub 4, the needle assembly 3 can be removed (e.g., via unscrewing) by rotating the housing 9 relative to the injection device 2.

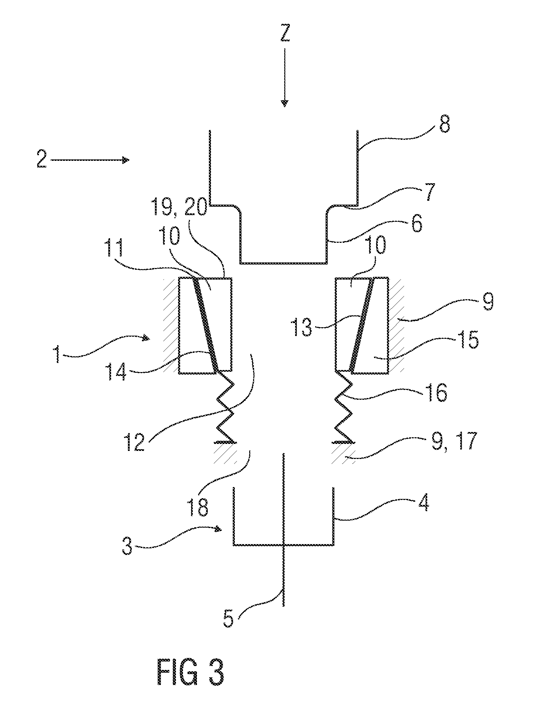

As shown in the exemplary embodiment in Figure 3, after the needle assembly 3 has been removed from the injection device 2, the needle assembly removal device 1 may be removed from the injection device 2. As the needle assembly removal device 1 is removed from the injection device 2, the spring 6 extends and the outer portion 15 moves in a second direction (e.g., distally) relative to the inner portion 10. As the outer portion 15 moves distally relative to the inner portion 10, the inner portion 10 moves radially outward, allowing the proximal opening 11 to return to the first width and thereby releasing the needle assembly 3. By orienting the proximal opening 11 or the distal opening 18 over a receptacle, the needle assembly 3 may be disposed of. The distal opening 18 may have a third width equal to or greater than the first width.

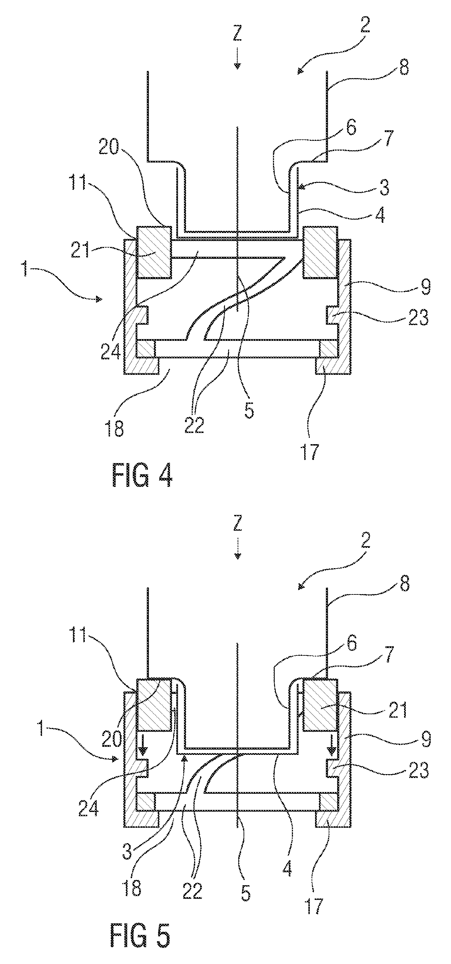

Figures 4-6 show another exemplary embodiment of a needle assembly removal device 1 according to the present invention. In this exemplary embodiment, the needle assembly removal device 1 includes a housing 9 with a clamping spring 22. The clamping spring 22 includes a proximal opening 11 and a distal opening 18. The proximal opening 11 of the clamping spring 22 may be sized and shaped to receive the needle hub 4 of the needle assembly 3. A slider 21 may be arranged on a proximal portion of the housing 9 and engage a proximal portion of the clamping spring 22. The slider 21 may be axially movably disposed in the housing 9.

As shown in the exemplary embodiment in Figure 4, after an injection has been administered, the needle assembly removal device 1 is aligned with the injection device 2 such that the needle hub 4 is aligned to be received in the proximal opening 11 of the clamping spring 22.

As shown in the exemplary embodiment in Figure 5, the needle assembly removal device 1 is advanced proximally until a proximal end 20 of the slider 21 abuts the shoulder portion 7 of the injection device 2. As the needle assembly removal device 1 is advanced further proximally, the clamping spring 22 compresses, and the slider 21 or a clamping section 24 of the clamping spring 22 engages the needle hub of the needle assembly 3. On or more stops 23 may be formed in the housing 9 to limit the movement of the slider 21 relative to the housing 9, or vice-versa. In this position, when the needle assembly 3 is engaged by the needle assembly removal device 1, the needle assembly 3 can be removed (e.g., via unscrewing) by rotating the housing 9 relative to the injection device 2.

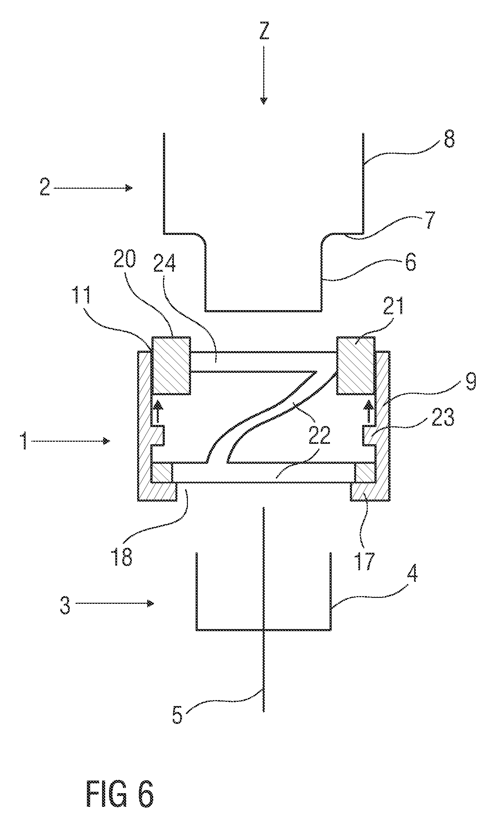

As shown in the exemplary embodiment in Figure 6, after the needle assembly 3 has been removed from the injection device 2, the needle assembly removal device 1 may be removed from the injection device 2. As the needle assembly removal device 1 is removed from the injection device 2, the clamping spring 6 extends and the slider 21 moves proximally relative to the housing 9. As the slider 21 moves proximally relative to the housing 9, the slider 21 or the clamping section 24 of the clamping spring 22 releases the needle assembly 3. By orienting the proximal opening 11 or the distal opening 18 over a receptacle, the needle assembly 3 may be disposed of.

Those of skill in the art will understand the modifications (additions and/or removals) of various components of the device and/or system and embodiment described herein may be made without departing from the full scope and spirit of the present invention, which encompass such modifications and any and all equivalents thereof.

### List of References

- 1: needle assembly removal device
- 2: injection device
- 3: needle assembly
- 4: needle hub
- 5: needle
- 6: neck portion
- 7: shoulder portion
- 8: body portion
- 9: housing
- 10: inner portion
- 11: proximal opening
- 12: channel
- 13: first angled surface
- 14: second angled surface
- 15: outer portion
- 16: spring
- 17: distal end
- 18: distal opening
- 19: proximal end
- 20: proximal end
- 21: slider
- 22: clamping spring
- 23: stop
- 24: clamping section
- z: direction

## Claims

1. A needle assembly removal device (1) comprising:
an inner portion (10);
a channel (12) formed in the inner portion (10), the channel (12) having a proximal opening (11);
an outer portion (15) movably disposed on the inner portion (10),
wherein movement of the outer portion (15) relative to the inner portion (10) in a first direction causes the inner portion (10) to change the proximal opening (11) from a first width to a second width.

2. The needle assembly removal device (1) according to claim 1, wherein the first width is greater than a diameter of a needle hub (4) of a needle assembly (3).

3. The needle assembly removal device (1) according to claim 2, wherein the second width is substantially equal to the diameter of the needle hub (4) of the needle assembly (3).

4. The needle assembly removal device (1) according to any one of the preceding claims, wherein the inner portion (10) includes a first angled surface (13) and the outer portion (15) includes a second angled surface (14), wherein the first angled surface (13) abuts the second angled surface (14).

5. The needle assembly removal device (1) according to any one of the preceding claims, wherein movement of the outer portion (15) relative to the inner portion (10) in a second direction causes the inner portion (10) to change the proximal opening (11) from the second width to the first width.

6. The needle assembly removal device (1) according to any one of the preceding claims, wherein the channel (12) includes a distal opening (12).

7. The needle assembly removal device (1) according to any one of the preceding claims, wherein a third width of the distal opening (12) is equal to or greater than the first width.

8. The needle assembly removal device (1) according to any one of the preceding claims, further comprising:
a spring (16) coupled to the inner portion (10).

9. The needle assembly removal device (1) according to claim 8, wherein the spring (16) is compressed during movement of the outer portion (15) relative to the inner portion (10) in the first direction.
